# EUROPÄISCHE PATENTSCHRIFT

(11) **EP 3 811 914 B1**
(45) Veröffentlichungstag und Bekanntmachung des Hinweises auf die Patenterteilung: **13.03.2024**
(21) Anmeldenummer: 20190760.7
(22) Anmeldetag: 12.08.2020
(51) Int. Cl.: A61F 9/007, A61B 17/34

(54) **VORRICHTUNG ZUM EINFÜHREN EINES INSTRUMENTS IN DAS MENSCHLICHE AUGE**
DEVICE FOR INSERTING AN INSTRUMENT INTO THE HUMAN EYE
DISPOSITIF PERMETTANT D'INSERER UN INSTRUMENT DANS L' IL HUMAIN

(30) Priorität: 24.10.2019 DE 102019216402
(43) Veröffentlichungstag der Anmeldung: 28.04.2021
(73) Patentinhaber: Geuder AG, 69126 Heidelberg (DE)
(72) Erfinder: Hattenbach, Lars-Olof, 67061 Ludwigshafen (DE); Kretz, Walter, 76703 Kraichtal (DE)
(74) Vertreter: Ullrich & Naumann PartG mbB

(56) Entgegenhaltungen:
- EP-A1- 1 693 011
- EP-A1- 2 036 506
- WO-A1-2011/037941
- DE-A1-102009 032 186
- DE-A1-102015 218 958

## Beschreibung

Die Erfindung betrifft eine Vorrichtung zum Einführen eines Instruments in das menschliche Auge mit einem Sondenkopf und einer sich daran anschließenden Sonde, wobei die Sonde zum Einstecken in das Auge und der Sondenkopf auf der der Sonde zugewandten Seite zur Anlage außerhalb des Auges dient, wobei sich ein Durchgang durch den Sondenkopf und die Sonde hindurch erstreckt, so dass ein Instrument über den Durchgang in das Auge einbringbar ist. Ophthalmologische Vorrichtungen der gattungsbildenden Art sind unter dem Begriff Inzisionsvorrichtung oder Trokar hinlänglich aus der Praxis bekannt. Lediglich beispielhaft sei dazu auf die EP 1 886 653 A1, DE 10 2015 218958 A1 und WO 2011/037941 A1 verwiesen.

Im Rahmen von ophthalmologischen Eingriffen werden regelmäßig mehrere Trokare durch die Sklera hindurch in das Auge eingeführt. Dabei werden oftmals ein oder zwei Trokare in das Auge eingesetzt, über die die benötigten Instrumente, beispielsweise Ultraschallschneidinstrumente oder Beleuchtungseinrichtungen, in das Auge einführbar sind. Diese Trokare werden üblicherweise als Instrumententrokare oder - entsprechend der Anwendung - als Beleuchtungstrokare bezeichnet.

Die hier in Rede stehenden Trokare haben den Vorteil, dass diese in eine kleine Öffnung der Sklera eingesetzt werden und dass die Instrumente durch den jeweiligen Trokar hindurch austauschbar sind. Eine weiterreichende Reizung oder Beschädigung der Sklera wird durch die Verwendung des Trokars vermieden.

Für den in das Auge eingesetzten Trokar gibt es grundsätzlich zwei Situationen. Eine Situation resultiert daraus, dass der Trokar - zunächst ohne Instrument - durch die Sklera hindurch in das Auge eingesetzt ist. Hier besteht die Gefahr, dass Flüssigkeit aus dem Auge heraus durch den im Trokar ausgebildeten Durchgang hindurch nach außen gelangt. Dies gilt insbesondere dann zu vermeiden, wenn von einer anderen Stelle her beispielsweise Spülflüssigkeit dem Auge zugeführt wird. Folglich gilt es den "leeren" Trokar weitestgehend abzudichten.

Eine andere Situation des Trokars ergibt sich bei eingesetztem Instrument. Auch hier besteht die Gefahr, dass zwischen der Wandung des Durchgangs und dem Instrument Flüssigkeit nach Außen gelangt, nämlich dann, wenn der Außendurchmesser des Instruments kleiner ist als der im Trokar ausgebildete Durchgang. Auch in dieser Situation ist eine Abdichtung erforderlich.

Aus der Praxis ist es bekannt, eine Dichtkappe aus Silikon an dem Sondenkopf anzubringen, um den Augeninnendruck aufrecht zu erhalten. Durch diesen wird das Instrument durchgesteckt, wobei die Dichtkappe derart eng an dem Instrument anliegt, dass auch bei eingebrachtem Instrument eine Abdichtung des Augeninneren erreicht wird, so dass der Augeninnendruck aufrechterhalten werden kann.

Die bekannten zweiteiligen Vorrichtungen sind aufwändig in der Herstellung, zumal der Trokar regelmäßig aus einem anderen Material besteht als die aus Silikon hergestellte Dichtkappe. Des Weiteren ist die Dichtkappe als solche schwierig zu fertigen, da diese gewährleisten muss, dass eine ausreichende Abdichtung erfolgt, unabhängig davon, ob ein Instrument eingeführt ist oder nicht. Daher muss die Fertigung mit entsprechend geringen Toleranzen bei einem ohnehin kleinen Bauteil erfolgen.

Der vorliegenden Erfindung liegt daher die Aufgabe zugrunde, eine Vorrichtung der eingangs genannten Art derart auszugestalten und weiterzubilden, dass mit konstruktiv einfachen Mitteln eine ausreichende Abdichtung des Augeninneren gewährleistet ist.

Erfindungsgemäß wird die voranstehende Aufgabe durch die Merkmale des Anspruches 1 gelöst. Danach ist die in Rede stehende Vorrichtung zum Einführen eines Instruments in das menschliche Auge mit einem Sondenkopf und einer sich daran anschließenden Sonde, wobei die Sonde zum Einstecken in das Auge und der Sondenkopf auf der der Sonde zugewandten Seite zur Anlage außerhalb des Auges dient, wobei sich ein Durchgang durch den Sondenkopf und die Sonde hindurch erstreckt, so dass ein Instrument über den Durchgang in das Auge einbringbar ist, wobei an der Sonde mindestens eine Öffnung ausgebildet ist, so dass bei in das Auge eingeführter Sonde Augengewebe in die Öffnung hineindrückt und den Durchgang verschließt. Die Sonde weist zwei sich in Längsrichtung der Sonde erstreckende Ausschnitte auf, wobei sich die Ausschnitte bis zu dem freien Ende der Sonde hin erstrecken.

In erfindungsgemäßer Weise ist zunächst erkannt worden, dass eine zweiteilige Ausgestaltung der Vorrichtung nicht notwendig ist, wenn die Abdichtungsmaßnahme über die Sonde erfolgt. Hierzu ist in der Sonde mindestens eine Öffnung vorgesehen, die derart ausgebildet ist, dass sich umliegendes Gewebe in die Öffnung hineingedrückt, wenn die Sonde innerhalb des Auges angeordnet ist. Durch das in die Öffnung eindringende Augengewebe wird eine hinreichende Abdichtung geschafften, so dass der Augeninnendruck auch aufrechterhalten wird, wenn kein Instrument in die Sonde eingeführt ist. Durch das Einführen des Instruments wird das Augengewebe durch die Öffnungen zurückgedrängt, wobei sich in dem Bereich zwischen dem Instrument und dem Sondeninneren weiterhin Gewebe befindet, welches als Dichtung wirkt. Somit wird auch bei eingeführtem Instrument ein Druckabfall verhindert. Durch die erfindungsgemäße Lehre ist es somit möglich, die Vorrichtung einteilig auszubilden, so dass diese in der Herstellung sowie in der Handhabung erheblich vereinfacht ist.

Der Begriff "Instrument" ist dabei im weitesten Sinne zu verstehen. Hierbei kann es sich um ein Ultraschallinstrument, eine Beleuchtungseinrichtung, eine Phakoemulsifikationsnadel oder ein beliebiges anderes Objekt handeln, das im Rahmen eines ophthalmologischen Eingriffs durch einen Trokar in das Auge eingeführt wird.

In vorteilhafter Weise könnte die Öffnung derart angeordnet sein, dass wenn der Sondenkopf außerhalb des Auges anliegt, die Sklera in die Öffnung hineindrückt. Aufgrund der Gewebeeigenschaften der Sklera ist diese in idealer Weise dazu geeignet, im Sinne eines Dichtmaterials zu wirken. Weiterhin ist die Sklera relativ robust, so dass eine Verletzung oder übermäßige Reizung des Gewebes nicht erfolgt.

Im Hinblick auf eine besonders zuverlässige Abdichtung ist es denkbar, dass mehrere Öffnungen ausgebildet sind. Im Konkreten könnten die Öffnungen in Umfangsrichtung der Sonde symmetrisch oder asymmetrisch angeordnet sein.

In weiter vorteilhafter Weise könnten die Öffnungen im gleichen Abstand zum Sondenkopf ausgebildet sein. Dadurch wird erreicht, dass das Gewebe symmetrisch auf ein eingeführtes Instrument drückt, so dass sich die Handhabung des Instruments verbessert.

Im Hinblick auf eine Anpassung an die bei einem Eingriff verwendeten Instrumente und/oder die bei dem Eingriff aufgrund der Handhabung der Instrumente auftretenden Kräfte, könnten zumindest zwei Öffnungen einen unterschiedlichen Abstand zum Sondenkopf aufweisen. Somit könnte die Anordnung der Öffnungen in Abstimmung auf die Verwendung der Vorrichtung optimiert werden.

In besonders vorteilhafter Weise könnten die Öffnung/Öffnungen im dem Sondenkopf zugewandten Drittel der Sonde ausgebildet sein. Dies hat den Vorteil, dass gewährleistet werden kann, dass lediglich die Sklera in die Öffnungen hineingedrückt wird.

Zur weiteren Vereinfachung der Handhabung und zur Minimierung von Traumata ist es denkbar, dass die Öffnung/Öffnungen auf der dem freien Ende der Sonde zugewandten Seite schräg ausgebildet bzw. eine Schräge aufweisen, so dass bei einem Herausziehen aus dem Auge das Augengewebe leichter aus der/den Öffnung/Öffnungen ausführbar ist. Eine entsprechende Schräge an der Öffnung dient somit als Ausführhilfe.

In besonders vorteilhafter Weise könnten der Außendurchmesser der Sonde und die Größe und Form der Öffnung/Öffnungen derart dimensioniert sein, dass die Bindehaut nicht in die Öffnung/Öffnungen gelangt, wenn der Sondenkopf außerhalb des Auges anliegt. Dadurch kann vermieden werden, dass die empfindliche Bindehaut in die Öffnungen eingedrückt und dabei gereizt bzw. verletzt wird.

Erfindungsgemäss weist die Sonde zwei sich in Längsrichtung erstreckende Ausschnitte auf. Bei der Anordnung von zwei Ausschnitten ist die Vorrichtung im Bereich der Sonde gabelartig ausgebildet. Dadurch wird der Vorteil erzielt, dass eine federnde Wirkung auf das Instrument, beispielsweise eine Trokarlanze erreicht wird, wodurch sich der Halt der Vorrichtung an dem Instrument erhöht. Des Weiteren ist durch die federnde Wirkung das Einführen von Instrumenten mit unterschiedlichen Gaugegrößen bzw. Durchmessern möglich. Hierzu können die Ausschnitte derart ausgebildet und angeordnet sein, dass die Sonde sich im Durchmesser ausdehnen kann. Somit könnten beispielsweise Instrumente mit mindestens zwei unterschiedlichen Gaugegrößen mit der Vorrichtung verwendet werden. Um diesen Effekt zu maximieren, erstreckt sich der Ausschnitt bis zu dem freien Ende der Sonde hin.

Zur Erzielung eines besseren Halts der Vorrichtung in dem Auge könnte die Sonde, insbesondere in dem dem Sondenkopf zugewandten Drittel, ein Gewinde und/oder Drehriefen oder eine andere Struktur auf ihrer Oberfläche aufweisen.

Des Weiteren ist es denkbar, dass das freie Ende der Sonde eine Schneide aufweist und/oder spitz ausgebildet ist. Somit könnte die Vorrichtung ohne die Verwendung einer Trokarlanze in das Auge eingeführt werden. Alternativ oder zusätzlich könnte der Durchgang im Sondenkopf in Richtung der Sonde trichterförmig zulaufen, um eine Einführhilfe für das Instrument zu bilden.

Es wird darauf hingewiesen, dass die Offenbarung auch eine Vorrichtung zum Einführen eines Instruments in das menschliche Auge betrifft, mit einem Sondenkopf und einer sich daran anschließenden Sonde, wobei die Sonde zum Einstecken in das Auge und der Sondenkopf auf der der Sonde zugewandten Seite zur Anlage außerhalb des Auges dient, wobei sich ein Durchgang durch den Sondenkopf und die Sonde hindurch erstreckt, so dass ein Instrument über den Durchgang in das Auge einbringbar ist und wobei die Sonde mindestens einen, vorzugsweise zwei sich in Längsrichtung erstreckende Ausschnitte aufweist.

Eine solche Vorrichtung könnte die in den Ansprüchen 1 bis 9 sowie in der voranstehenden Beschreibung genannten Merkmale und Vorteile aufweisen.

Die zugrundeliegende Aufgabe wird des Weiteren durch die Merkmale des Anspruchs 10 gelöst. Damit ist ein Set bestehend aus einer Vorrichtung nach einem der Ansprüche 1 bis 9 und einem Instrument offenbart, wobei das Instrument einen derart auf die Sonde abgestimmten Wirkbereich aufweist, dass, wenn die Sonde in das Auge eingeführt ist, durch Einbringen des Wirkbereichs in die Sonde das Augengewebe aus der Öffnung oder den Öffnungen der Sonde ausbringbar ist.

Dabei ist erkannt worden, dass sich die erfindungsgemäße Vorrichtung auf besonders schonende Weise aus dem Auge entfernen lässt, indem ein Instrument mit einem Wirkbereich eingeführt wird, der auf die Sonde derart abgestimmt ist, dass das Gewebe aus der Öffnung bzw. den Öffnungen herausgedrückt wird. Bei dem Wirkbereich kann es sich beispielsweise um ein stabartiges Element handeln, dessen Außendurchmesser zumindest im Wesentlichen dem Innendurchmesser der Sonde entspricht.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigt
- Fig. 1: in einer schematischen, perspektivischen Darstellung ein Ausführungsbeispiel einer erfindungsgemäßen Vorrichtung,
- Fig. 2: in einer schematischen Darstellung eine seitliche Ansicht eines Instruments eines erfindungsgemäßen Sets.

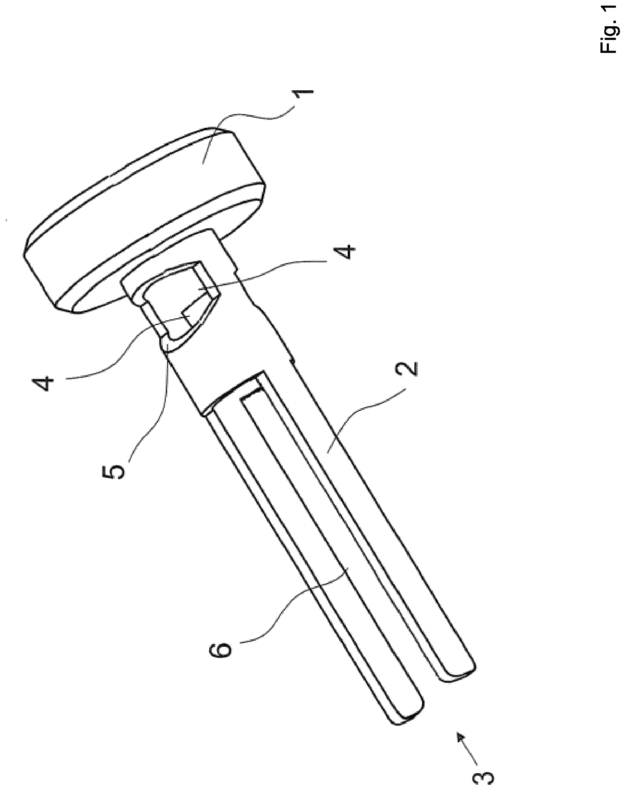

Figur 1 zeigt eine erfindungsgemäße Vorrichtung zum Einführen eines Instruments in das menschliche Auge. Die Vorrichtung umfasst einen Sondenkopf 1 und eine sich daran anschließende Sonde 2. Durch den Sondenkopf 1 und die Sonde 2 hindurch erstreckt sich ein Durchgang 3, über den das Instrument in das Auge einführbar ist. Der Sondenkopf 1 ist in diesem Ausführungsbeispiel flach ausgebildet, so dass das Risiko verringert wird, dass während des Eingriffs versehentlich der Sondenkopf 1 seitlich berührt wird, wodurch die Sonde 2 verschoben oder im Extremfall aus dem Auge herausgezogen würde.

An der Sonde 2 sind zwei Öffnungen 4 ausgebildet, durch die Gewebe, insbesondere die Sklera, in den Durchgang 3 hineingedrückt wird, wenn sich die Sonde 2 innerhalb des Auges befindet und der Sondenkopf 1 an dem Auge anliegt. Dadurch wird der Durchgang 3 derart verschlossen bzw. abgedichtet, dass der Augeninnendruck zumindest im Wesentlichen aufrechterhalten wird.

Dabei ist des Weiteren deutlich zu erkennen, dass an dem Bereich des Durchgangs 3, der dem freien Ende der Sonde 2 zugewandt ist, die Öffnung 4 eine Schräge 5 aufweist. Durch die Schräge 5 kann Gewebe leichter aus der Öffnung 4 herausgleiten, wenn die Sonde 2 aus dem Auge herausgezogen wird.

Weiterhin geht aus Figur 1 deutlich hervor, dass die Sonde 2 zwei Einschnitte 6 aufweist, die sich bis zu dem freien Ende der Sonde 2 hin erstrecken. Die Sonde 2 hat in diesem Bereich somit eine gabelartige Form. Aufgrund dieser Konstruktion ist die Sonde 2 im Bereich der Einschnitte 6 federnd ausgebildet, so dass Instrumente mit unterschiedlichen Gaugegrößen bzw. Durchmessern eingeführt werden können.

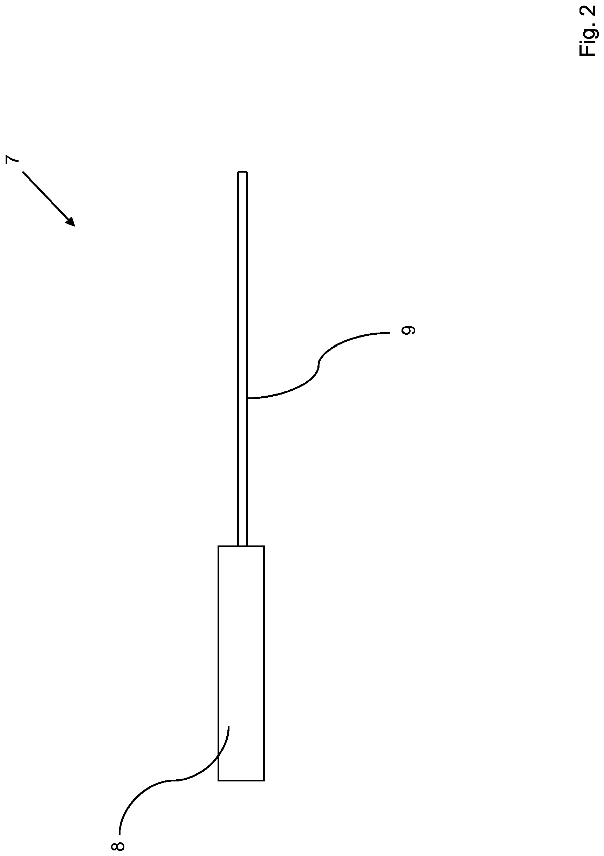

Figur 2 zeigt ein Instrument 7 eines erfindungsgemäßen Sets. Das Instrument 7 weist einen Griff 8 und einen sich daran anschließenden Wirkbereich 9 auf. Der Wirkbereich 9 ist stabartig ausgebildet und von seiner Abmessung her auf den Durchgang 3 der Sonde 2 abgestimmt. Somit kann durch Einschieben des Wirkbereichs 9 das Augengewebe aus den Öffnungen 4 gedrückt werden, so dass die Sonde 2 aus dem Auge herausnehmbar ist, ohne dieses zu verletzen.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Sondenkopf
- 2: Sonde
- 3: Durchgang
- 4: Öffnung
- 5: Schräge
- 6: Ausschnitt
- 7: Instrument
- 8: Griff
- 9: Wirkbereich

## Patentansprüche

1. Vorrichtung zum Einführen eines Instruments in das menschliche Auge mit einem Sondenkopf (1) und einer sich daran anschließenden Sonde (2), wobei die Sonde (2) zum Einstecken in das Auge und der Sondenkopf (1) auf der der Sonde (2) zugewandten Seite zur Anlage außerhalb des Auges dient, wobei sich ein Durchgang (3) durch den Sondenkopf (1) und die Sonde (2) hindurch erstreckt, so dass ein Instrument über den Durchgang (3) in das Auge einbringbar ist, wobei an der Sonde (2) mindestens eine Öffnung (4) ausgebildet ist, so dass bei in das Auge eingeführter Sonde (2) Augengewebe in die Öffnung (4) hineindrückt und den Durchgang (3) verschließt, wobei die Sonde (2) zwei sich in Längsrichtung der Sonde (2) erstreckende Ausschnitte (6) aufweist, und wobei sich die Ausschnitte (6) bis zu dem freien Ende der Sonde (1) hin erstrecken.

2. Vorrichtung nach Anspruch 1, **dadurch gekennzeichnet, dass** die Öffnung (4) derart angeordnet ist, dass, wenn der Sondenkopf (1) außerhalb des Auges anliegt, die Sklera in die Öffnung (4) hineindrückt.

3. Vorrichtung nach Anspruch 1 oder 2, **dadurch gekennzeichnet, dass** mehrere Öffnungen (4) ausgebildet sind.

4. Vorrichtung nach Anspruch 3, **dadurch gekennzeichnet, dass** die Öffnungen (4) in Umfangsrichtung der Sonde (2) symmetrisch oder asymmetrisch angeordnet sind.

5. Vorrichtung nach einem der Ansprüche 3 oder 4, **dadurch gekennzeichnet, dass** die Öffnungen (4) im gleichen Abstand zum Sondenkopf (1) ausgebildet sind.

6. Vorrichtung nach einem der Ansprüche 3 oder 4, **dadurch gekennzeichnet, dass** zumindest zwei Öffnungen (4) einen unterschiedlichen Abstand zum Sondenkopf (1) aufweisen.

7. Vorrichtung nach einem der Ansprüche 1 bis 6, **dadurch gekennzeichnet, dass** die Öffnung/Öffnungen (4) im dem Sondenkopf (1) zugewandten Drittel der Sonde (2) ausgebildet sind.

8. Vorrichtung nach einem der Ansprüche 1 bis 7, **dadurch gekennzeichnet, dass** die Öffnung/Öffnungen (4) auf der dem freien Ende der Sonde (2) zugewandten Seite schräg ausgebildet sind, so dass bei einem Herausziehen aus dem Auge das Augengewebe leichter aus der/den Öffnung/Öffnungen (4) ausführbar ist.

9. Vorrichtung nach einem der Ansprüche 1 bis 8, **dadurch gekennzeichnet, dass** der Außendurchmesser der Sonde (2) und die Größe und Form der Öffnung/Öffnungen (4) derart dimensioniert sind, dass die Bindehaut nicht in die Öffnung/Öffnungen (4) eindringt, wenn der Sondenkopf (1) außerhalb des Auges anliegt.

10. Set bestehend aus einer Vorrichtung nach einem der Ansprüche 1 bis 9 und einem Instrument (7), wobei das Instrument (7) einen derart auf die Sonde (2) abgestimmten Wirkbereich (9) aufweist, dass wenn die Sonde (2) in das Auge eingeführt ist, durch Einbringen des Wirkbereichs (9) in die Sonde (2) das Augengewebe aus der Öffnung (4) oder den Öffnungen (4) der Sonde (2) ausbringbar ist.

## Claims

1. Apparatus for introducing an instrument into the human eye, having a probe head (1) and a probe (2) which is adjacent thereto, wherein the probe (2) is used for insertion into the eye and the probe head (1) is used for abutment outside the eye at the side facing the probe (2), wherein a passage (3) extends through the probe head (1) and the probe (2) so that an instrument can be introduced via the passage (3) into the eye, wherein at least one opening (4) is formed on the probe (2) so that, when the probe (2) is inserted into the eye, eye tissue is pressed into the opening (4) and closes the passage (3), wherein the probe (2) has two cut-outs (6) which extend in the longitudinal direction of the probe (2), and wherein the cut-outs (6) extend up to the free end of the probe (1).

2. Apparatus according to claim 1, **characterised in that** the opening (4) is arranged in such a manner that, when the probe head (1) abuts outside the eye the sclera presses into the opening (4).

3. Apparatus according to claim 1 or 2, **characterised in that** a plurality of openings (4) are formed.

4. Apparatus according to claim 3, **characterised in that** the openings (4) are arranged symmetrically or asymmetrically in the circumferential direction of the probe (2).

5. Apparatus according to either claim 3 or claim 4, **characterised in that** the openings (4) are formed with the same spacing with respect to the probe head (1).

6. Apparatus according to either claim 3 or 4, **characterised in that** at least two openings (4) have a different spacing with respect to the probe head (1).

7. Apparatus according to any one of claims 1 to 6, **characterised in that** the opening/openings (4) is/are formed in the third of the probe (2) facing the probe head (1).

8. Apparatus according to any one of claims 1 to 7, **characterised in that** the openings/openings (4) at the side facing the free end of the probe (2) are constructed in an oblique manner so that, when pulled out of the eye, the eye tissue can be more easily guided out of the opening(s) (4).

9. Apparatus according to any one of claims 1 to 8, **characterised in that** the outer diameter of the probe (2) and the size and shape of the opening(s) (4) are sized in such a manner that the conjunctiva does not penetrate into the opening(s) (4) when the probe head (1) abuts outside the eye.

10. Set comprising an apparatus according to any one of claims 1 to 9 and an instrument (7), wherein the instrument (7) has an active region (9) which is adapted to the probe (2) in such a manner that, when the probe (2) is introduced into the eye, by introducing the active region (9) into the probe (2) the eye tissue can be removed from the opening (4) or the openings (4) of the probe (2).

## Revendications

1. Dispositif pour l'introduction d'un instrument dans l'œil humain avec une tête de sonde (1) et une sonde (2) qui s'y raccorde, dans lequel la sonde (2) est destinée à être insérée dans l'œil et la tête de sonde (1) est destinée à s'appuyer contre la face externe orientée vers la sonde (2), à l'extérieur de l'œil, dans lequel un passage (3) s'étend à travers la tête de sonde (1) et la sonde (2), de sorte qu'un instrument peut être introduit dans l'œil à travers le passage (3), dans lequel, au niveau de la sonde (2) est réalisée au moins une ouverture (4) de sorte que, lorsque la sonde (2) est introduite dans l'œil, le tissu de l'œil s'enfonce dans l'ouverture (4) et ferme le passage (3), dans lequel la sonde (2) comprend deux découpes (6) s'étendant dans la direction longitudinale de la sonde (2) et dans lequel les découpes (6) s'étendant jusqu'à l'extrémité libre de la sonde (1).

2. Dispositif selon la revendication 1, **caractérisé en ce que** l'ouverture (4) est disposée de sorte que, lorsque la tête de sonde (1) s'appuie à l'extérieur de l'œil, la sclérotique s'enfonce dans l'ouverture (4).

3. Dispositif selon la revendication 1 ou 2, **caractérisé en ce que** plusieurs ouvertures (4) sont réalisées.

4. Dispositif selon la revendication 3, **caractérisé en ce que** les ouvertures (4) sont disposées de manière symétrique ou asymétrique dans la direction de la circonférence de la sonde (2).

5. Dispositif selon l'une des revendications 3 ou 4, **caractérisé en ce que** les ouvertures (4) sont réalisées à la même distance de la tête de sonde (1).

6. Dispositif selon l'une des revendications 3 ou 4, **caractérisé en ce qu'**au moins deux ouvertures (4) présentent une distance différente par rapport à la tête de sonde (1).

7. Dispositif selon l'une des revendications 1 à 6, **caractérisé en ce que** l'ouverture ou les ouvertures (4) sont réalisées dans le tiers de la sonde (2) orienté vers la tête de sonde (1).

8. Dispositif selon l'une des revendications 1 à 7, **caractérisé en ce que** l'ouverture ou les ouvertures (4) sont réalisées de manière inclinée sur le côté orienté vers l'extrémité libre de la sonde (2), de sorte que, lors d'une extraction hors de l'œil, le tissu de l'œil peut être évacué plus facilement de l'ouverture ou des ouvertures (4).

9. Dispositif selon l'une des revendications 1 à 8, **caractérisé en ce que** le diamètre extérieur de la sonde (2) et la taille et la forme de l'ouverture ou des ouvertures (4) sont dimensionnés de sorte que la conjonctive ne pénètre pas dans l'ouverture ou les ouvertures (4) lorsque la sonde (1) s'appuie à l'extérieur de l'œil.

10. Kit constitué d'un dispositif selon l'une des revendications 1 à 9 et d'un instrument (7), dans lequel l'instrument (7) présente une partie active (9) adaptée à la sonde (2), de sorte que lorsque la sonde (2) est introduite dans l'œil, grâce à l'introduction de la partie active (9) dans la sonde (2), le tissu de l'œil peut être évacué par l'ouverture (4) ou les ouvertures (4) de la sonde (2).
